# EUROPÄISCHE PATENTANMELDUNG

(11) **EP 1 813 591 A1**
(43) Veröffentlichungstag der Anmeldung: **01.08.2007**
(21) Anmeldenummer: 07000855.2
(22) Anmeldetag: 17.01.2007
(51) Int. Cl.: C07C 51/083, C07C 55/30

(54) **Bicyclo[4.3.0] nonan-3(4),7(8)-dicarbonsäure und ein Verfahren zu ihrer Herstellung**

(30) Priorität: 31.01.2006 DE 10604319
(71) Anmelder: OXEA Deutschland GmbH, 46147 Oberhausen (DE)
(72) Erfinder: Bavaj, Paolo, 60320 Frankfurt (DE); Springer, Helmut, 46539 Dinslaken (DE)

(57) **Zusammenfassung**

Die vorliegende Erfindung betrifft die chemische Verbindung Bicyclo[4.3.0]nonan-3(4), 7(8)-dicarbonsäure sowie ein Verfahren zu ihrer Herstellung, wobei man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt und das so erhaltene 3(4),7(8)-Bisformyl-bicyclo[4.3.0]nonan oxidiert oder zunächst zum 3(4),7(8)-Dihydroxymethyl-bicyclo[4.3.0]nonan hydriert und das so erhaltene Diol in einer Alkalischmelze umsetzt.

## Beschreibung

Die vorliegende Erfindung betrifft Bicyclo[4.3.0]nonan-3(4),7(8)-dicarbonsäure und ein Verfahren zu ihrer Herstellung aus Bicyclo[4.3.0]nona-3,7-dien.

Kondensierte, alicyclische, ungesättigte Kohlenwasserstoffe mit isolierten Doppelbindungen in den Ringen sind wertvolle Ausgangsprodukte, die sich zu anwendungstechnisch wichtigen Verbindungen umsetzen lassen. Das ringförmig aufgebaute und kondensierte Kohlenwasserstoffgerüst verleiht dabei besondere Eigenschaften. Ein wichtiges Beispiel für diese Verbindungsklasse ist das durch Dimerisierung von Cyclopentadien leicht zugängliche und auch großtechnisch hergestellte Dicyclopentadien (DCP), das sich zu anwendungstechnisch wichtigen Verbindungen umsetzen lässt, denen das Tricyclodecan-Gerüst besondere Eigenschaften verleiht. Die vom DCPabgeleiteten Verbindungen mit Tricyclodecan-Struktur werden in der Literatur häufig auch als TCD-Derivate bezeichnet (Chemiker-Zeitung, 98, 1974, Seiten 70 bis 76).

Insbesondere die Hydroformylierung von DCP liefert interessante TCD-Aldehyde, wie 3(4),8(9)-Bisformyl-tricyclo[5.2.1.0^{2.6}]decan, auch als TCD-Dialdehyd bezeichnet, das zu wichtigen Zwischenprodukten weiterverarbeitet wird. Wegen ihrer thermischen Labilität, die bei der destillativen Aufarbeitung zu Verlusten führt, wird TCD-Dialdehyd zumeist nicht rein isoliert, sondern als Rohprodukt der Hydroformylierungsreaktion weiterverarbeitet.

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Kobalt als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄-ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Aufgrund der großen Bedeutung der Hydroformylierungsprodukte des DCP finden sich auch in der technischen Literatur zahlreiche Arbeiten, die sich sowohl mit der Hydroformylierungsreaktion von DCP als auch mit der nachfolgenden Aufarbeitung des Rohproduktes befassen. So behandeln DE 38 22 038 A1 und GB 1 170 226 die Hydroformylierung von DCP in Gegenwart von Rhodium in einem organischen Lösungsmittel bei erhöhtem Druck und erhöhter Temperatur. Eine zusammenfassende Darstellung über die Hydroformylierung von Dicyclopentadien findet sich in der Chemiker-Zeitung 98, 1974, 70-76, wobei ebenfalls auf die thermische Labilität der TCD-Aldehyde hingewiesen wird, die bei der destillativen Aufarbeitung des rohen Hydroformylierungsgemisches zu hohen Produktverlusten führt. Daher werden TCD-Dialdehyde zumeist nicht rein isoliert, sondern in ihren Gemischen mit den Nebenprodukten der Oxo-Synthese weiterverarbeitet. Es finden sich im Stand der Technik, z.B. in EP-1 065 194 A1 oder US 5,138,101 A aber auch Hinweise auf extraktive Aufarbeitungsprozesse ohne thermische Belastung. Dabei wird das organische Rohgemisch mit einem polaren organischen Lösungsmittel, beispielsweise mit einem mehrwertigen Alkohol oder mit einem Methanol/Wasser-Gemisch extrahiert, wobei die TCD-Dialdehyde in die polare, alkoholische Phase übergehen und der Hydroformylierungskatalysator in der Kohlenwasserstoffphase verbleibt.

TCD-Dialdehyde werden zu wichtigen Zwischenprodukten weiterverarbeitet. So führt die Oxidation von TCD-Dialdehyd zu Tricyclo[5.2.1.0^{2.6}]decan-3(4),8(9)-dicarbonsäure, auch als TCD-Säure DS bezeichnet, die als wertvolles Zwischenprodukt für die chemische Industrie eine große wirtschaftliche Bedeutung besitzt (J.Prakt. Chem. 14, (1961), 71; Chemiker-Zeitung 98, 1974, Seiten 70-76).

Ein weiterer Zugang zur Dicarbonsäure eröffnet sich über die Alkalischmelze des aus dem durch Hydrierung von TCD-Dialdehyd gewonnenen TCD-Alkohol DM {3(4),8(9)-Dihydroxymethyl-tricyclo[5.2.1.0².⁶]decan}. Die Alkalischmelze erfolgt durch Umsetzung des Alkohols mit geschmolzenem Alkalimetallhydroxid bei erhöhter Temperatur und erhöhtem Druck und führt unter Wasserstoffentwicklung zu den Alkalimetallsalzen der Säure (US 2,881,208 A, US 2,875,244 A, FR 1,155,677 A).

Die zweiwertige TCD-Säure DS ist in vielfältiger Weise für unterschiedliche Anwendungen von hohem technischen Interesse, zum Beispiel als Säurekomponente in Lack- und Kunstharzformulierungen, für Weichmacher und in Schmierölen (US 2,875,244 A, US 2,881,208 A).

Aufgrund der großen wirtschaftlichen Bedeutung, die Dicarbonsäuren auf Basis von kondensierten, alicyclischen Kohlenwasserstoffen besitzen, besteht daher der Bedarf nach der Bereitstellung weiterer, preisgünstig verfügbarer Dicarbonsäuren in hoher Reinheit, die ein ringförmig aufgebautes Kohlenwasserstoffgerüst mit kondensierten Ringen aufweisen.

Die vorliegende Erfindung betrifft daher die chemische Verbindung Bicyclo[4.3.0]nonan-3(4),7(8)-dicarbonsäure.

Ebenfalls besteht die Erfindung in einem Verfahren zur Herstellung von Bicyclo[4.3.0]nonan-3(4),7(8)-dicarbonsäure durch Hydroformylierung von Bicyclo[4.3.0]nona-3,7-dien mit nachfolgender Oxidation. Es ist dadurch gekennzeichnet, dass man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt und das so erhaltene 3(4),7(8)-Bisformylbicyclo[4.3.0]nonan anschließend zur Bicyclo[4.3.0]nonan-3(4),7(8)-dicarbonsäure oxidiert.

Die Erfindung besteht weiterhin in einem Verfahren zur Herstellung von Bicyclo[4.3.0]nonan-3(4),7(8)-dicarbonsäure durch Hydroformylierung von Bicyclo[4.3.0]nona-3,7-dien mit nachfolgender Hydrierung und anschließender Umsetzung in einer Alkalischmelze. Es ist dadurch gekennzeichnet, dass man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt, das so erhaltene 3(4),7(8)-Bisformyl-bicyclo[4.3.0]nonan zum 3(4),7(8)-Dihydroxymethyl-bicyclo[4.3.0]nonan hydriert, das so erhaltene 3(4),7(8)-Dihydroxymethyl-bicyclo[4.3.0]nonan in einer Alkalischmelze bei erhöhter Temperatur und erhöhtem Druck umsetzt und anschließend Bicyclo[4.3.0]nonan-3(4),7(8)-dicarbonsäure freisetzt.

Die erfindungsgemäße Verbindung leitet sich von Bicyclo[4.3.0]nona-3,7-dien ab, das technisch durch Diels-Alder-Reaktion von Butadien mit Cyclopentadien hergestellt wird und das daher in preisgünstigen Mengen zur Verfügung steht.

Die Zählung der in dem ungesättigten, bicyclischen Kohlenwasserstoff gebundenen Kohlenstoffatome erfolgt nach folgender Reihenfolge: 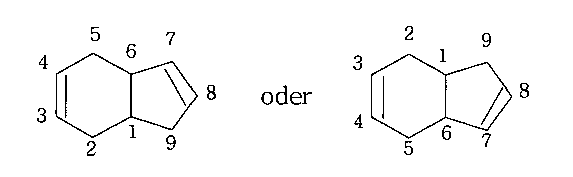 wobei beide Strukturformeln identisch sind.

Bei der erfindungsgemäßen Verbindung Bicyclo[4.3.0]nonan-3(4),7(8)-dicarbonsäure handelt es sich um ein Gemisch aus verschiedenen Isomeren der Bicyclo[4.3.0]nonan-dicarbonsäure, bei denen die Carboxylgruppe im Sechsring einmal an der 3- oder an der 4-Position und die Carboxylgruppe im Fünfring einmal an der 7- oder an der 8-Position gebunden sein kann.

In Analogie zu der bei den TCD-Derivaten gemäß Chemiker-Zeitung, 98, 1974, Seiten 70 bis 76 üblichen Schreibweise kann die erfindungsgemäße Verbindung Bicyclo[4.3.0]nonan-3(4),7(8)-dicarbonsäure formelmäßig wie folgt beschrieben werden: 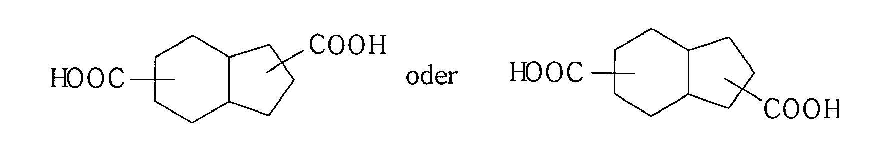 wobei beide Strukturformeln identisch sind.

Das Ausgangsprodukt Bicyclo[4.3.0]nona-3,7-dien kann als solches oder in Lösung der Hydroformylierung zugeführt werden. Geeignete Lösungsmittel sind solche, in denen Ausgangsmaterial, Reaktionsprodukt und Katalysator löslich sind und die sich unter den Reaktionsbedingungen inert verhalten, beispielsweise wasserunlösliche Ketone, Dialkylether, aliphatische Nitrile, aromatische Kohlenwasserstoffe wie Benzol, Toluol, die isomeren Xylole oder Mesitylen und gesättigte cycloaliphatische Kohlenwasserstoffe wie Cyclopentan oder Cylcohexan oder gesättigte aliphatische Kohlenwasserstoffe, wie n-Hexan, n-Heptan oder n-Octan. Der Anteil des Lösungsmittels im Reaktionsmedium kann über einen weiten Bereich variiert werden und beträgt üblicherweise zwischen 10 und 80 Gew.-%, vorzugsweise 20 bis 50 Gew.-%, bezogen auf das Reaktionsgemisch.

Die Hydroformylierungsstufe führt man in einem homogenen Reaktionssystem durch. Der Begriff homogenes Reaktionssystem steht für eine im wesentlichen aus Lösungsmittel, falls in der Reaktionsstufe zugesetzt, Katalysator, überschüssiger Organophosphorverbindung, unumgesetzter Ausgangsverbindung und Hydroformylierungsprodukt zusammengesetzte homogene Lösung.

Als Katalysatoren verwendet man Übergangsmetallverbindungen der Gruppe VIII des Periodensystems der Elemente, die Organophosphorverbindungen in komplexer Bindung enthalten. Vorzugsweise setzt man Komplexverbindungen des Kobalts, Rhodiums, Iridiums, Nickels, Eisens, Platins, Palladiums oder Rutheniums und insbesondere des Kobalts, Rhodiums und Iridiums ein. Besonders bevorzugt ist die Verwendung von Rhodium-Komplexverbindungen, die organische Phosphor(III)-Verbindungen als Liganden enthalten. Derartige Komplexverbindungen und ihre Herstellung sind bekannt (z.B. aus US 3 527 809 A, US 4 148 830 A, US 4 247 486 A, US 4 283 562 A). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von 5 bis 1000 Gew.-ppm und beträgt vorzugsweise 10 bis 700 Gew.-ppm. Insbesondere wendet man Rhodium in Konzentrationen von 20 bis 500 Gew.-ppm, jeweils bezogen auf das homogene Reaktionsgemisch an. Die Hydroformylierung wird in Gegenwart eines Katalysatorsystems aus Rhodium-Organophosphor-Komplexverbindung und freiem, d.h. überschüssigen Organophosphor-Liganden durchgeführt, der mit Rhodium keine Komplexverbindung mehr eingeht. Der freie Organophosphor-Ligand kann der gleiche sein wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Organophosphor-Komplexverbindungen, die als Katalysatoren Anwendung finden können, sind in US 3 527 809 A beschrieben. Zu den bevorzugten Liganden in den Rhodium-Komplexkatalysatoren zählen z. B. Triarylphosphine wie Triphenylphosphin, Trialkylphosphine wie Tri-(n-octyl)-phosphin, Trilaurylphosphin, Tri-(cyclohexyl)-phosphin, Alkylarylphosphine, Alkylphosphite, Arylphosphite, Alkyldiphosphite und Aryldiphosphite. So können Rhodium-Komplexverbindungen, die Arylphosphite der allgemeinen Formel P(OR¹)(OR²)(OR³) komplexgebunden enthalten, wobei wenigstens eine der Gruppen R¹, R² oder R³ für einen in ortho-Position substituierten Phenylring steht, ebenfalls eingesetzt werden. Als geeignete Komplexliganden haben sich Tris(2-tertiärbutylphenyl)phosphit oder Tris(2-tertiär-butyl-4-methylphenyl)phosphit erwiesen. Die Rhodium katalysierte Hydroformylierung von Olefinen mit Phosphit modifizierten Komplexverbindungen ist aus EP 0 054 986 A1 bekannt. Wegen seiner leichten Zugänglichkeit wird Triphenylphosphin besonders häufig angewandt.

Üblicherweise beträgt in der homogenen Reaktionsmischung das molare Verhältnis von Rhodium zu Phosphor 1 : 5 bis 1 : 200, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 10 bis 1 : 100 ein.

Verwendet man in der Hydroformylierungsstufe ein anderes Übergangsmetall der Gruppe VIII des Periodensystems der Elemente als Rhodium, so liegt die Konzentration an Übergangsmetall und das molare Verhältnis von Übergangsmetall zu Phosphor in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Die Bedingungen, unter denen die Hydroformylierung abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung von Bicyclo[4.3.0]nona-3,7-dien bei Temperaturen von 70 bis 160°C durch. Bevorzugt hält man Temperaturen von 80 bis 150°C und insbesondere von 90 bis 140°C ein. Der Gesamtdruck erstreckt sich über einen Bereich von 5 bis 35 MPa, vorzugsweise 10 bis 30 MPa und insbesondere 20 bis 30 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Übergangsmetall oder Übergangsmetallverbindung, Organophosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Zur Herstellung des Hydroformylierungskatalysators gelangt das Übergangsmetall der Gruppe VIII des Periodensystems der Elemente, insbesondere Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie Übergangsmetall-2-Ethylhexanoate, -Acetate, -Oxalate, -Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆, C_{O2}(CO)ₐ, Co₄(CO)₁₆, Fe(CO)₅, Fe₂(CO)₉, Ir₂(CO)₈, Ir₄(CO)₁₂ oder Übergangsmetall-Komplexverbindungen, z.B. Cyclopentadienylrhodiumverbindungen, Rhodiumacetylacetonat, Cyclopentadienylkobalt-Cyclooctadien-1,5, Fe(CO)₃-Cyclooctadien-1,5, [RhCl(Cyclooctadien-1,5]₂ oder PtCl₂(Cyclooctadien-1,5) eingesetzt werden. Übergangsmetallhalogenverbindungen kommen wegen ihres korrosiven Verhaltens der Halogenidionen weniger in Betracht.

Bevorzugt werden Übergangsmetalloxide und insbesondere Übergangsmetallacetate und -2-ethylhexanoate. Als besonders geeignet haben sich Rhodiumoxid, Rhodiumacetat, Rhodium-2-ethylhexanoat, Kobaltoxid, Kobaltacetat und Kobalt-2-ethylhexanoat erwiesen.

Die Hydroformylierungsstufe kann sowohl absatzweise als auch kontinuierlich durchgeführt werden. Nach dem erfindungsgemäßen Verfahren wird das Ausgangsolefin Bicyclo[4.3.0]nona-3,7-dien nahezu vollständig umgesetzt und es wird ein rohes Hydroformylierungsprodukt mit einem hohen Gehalt an dem gewünschten Bisformylprodukt erhalten, der im allgemeinen über 75 Gew.-%, bezogen auf das rohe Hydroformylierungsprodukt, liegt.

Das Umsetzungsprodukt der Hydroformylierungsstufe wird im allgemeinen ohne weitere Reinigung und ohne Katalysatorabtrennung weiterverarbeitet, zweckmäßigerweise schließt sich jedoch eine destillative Reinigung des Dialdehyds vor der Weiterverarbeitung an.

In einer Ausführungsform des erfindungsgemäßen Verfahrens erfolgt die Umsetzung der zweckmäßigerweise aufgereinigten Aldehyde mit Sauerstoff oder Sauerstoff enthaltenden Gasen oder in Gegenwart eines üblichen Oxidationsmittels, beispielsweise Wasserstoffperoxid, Alkalihypochlorite oder Kaliumpermanganat. Bevorzugt ist die Verwendung von Sauerstoff oder Sauerstoff enthaltenden Gasen sowohl in Abwesenheit als auch in Anwesenheit von Oxidationskatalysatoren.

Weitere Bestandteile derartiger Gasgemische sind inerte Gase, z.B. Stickstoff, Edelgase und Kohlendioxid. Der Anteil der inerten Bestandteile des Sauerstoff enthaltenden Gasgemisches beträgt bis zu 90 Vol-%, insbesondere 30 bis 80 Vol.-%. Die bevorzugten Oxidationsmittel sind Sauerstoff oder Luft.

Als Katalysatoren für den Oxidationsschritt kommen vorwiegend Salze von Übergangsmetallen in Betracht, insbesondere Salze des Kobalts und des Mangans sowie des Chroms, Eisens, Kupfers, Nickels, Silbers und Vanadiums, wie beispielsweise aus DE 100 10 771 C1 bekannt. Auch kann sich ein Zusatz von Alkalimetallsalzen schwacher Säuren vorteilhaft auf die Selektivität zur gewünschten Dicarbonsäure auswirken.

Die Aldehyde werden gelöst in einem unter den Reaktionsbedingungen inerten Lösungsmittel eingesetzt. Die Zugabe eines Lösungsmittels ist erforderlich, da die bei der Oxidation gebildete Bicyclo[4.3.0]nonan-3(4),7(8)-dicarbonsäure bei Raumtemperatur fest ist. Beispiele für geeignete Lösungsmittel sind Ketone wie Aceton, Ester, z.B. Ethylacetat, Kohlenwasserstoffe, z.B. Toluol und Nitrokohlenwasserstoffe wie Nitrobenzol. Die Konzentration des Aldehyds wird durch seine Löslichkeit sowie durch die Löslichkeit der gebildeten Dicarbonsäure in dem Lösungsmittel begrenzt.

Der Oxidationsschritt kann absatzweise oder kontinuierlich durchgeführt werden. Eine Rückführung nicht umgesetzter Reaktionsteilnehmer ist in beiden Fällen möglich.

Nach einer bewährten Ausführungsform legt man 3(4),7(8)-Bisformylbicyclo[4.3.0]nonan zusammen mit einem Lösungsmittel, beispielsweise Toluol, in einem geeigneten Reaktor, z.B. in einem mit einem Anströmboden versehenen Rohrreaktor, der gegebenenfalls noch Füllkörper enthält, vor und leitet den Sauerstoff oder das Sauerstoff enthaltende Gasgemisch von unten durch den Aldehyd. Die Umsetzung der Reaktionspartner erfolgt in einem Temperaturbereich von 20 bis 80°C, vorzugsweise zwischen 40 bis 80°C, bei Normaldruck. Die Anwendung erhöhten Drucks ist jedoch nicht ausgeschlossen. Üblicherweise arbeitet man in einem Bereich von Normaldruck bis 1,0 MPa, vorzugsweise bei Normaldruck bis 0,8 MPa.

Entsprechend einer weiteren Ausführungsform verwendet man als Reaktor einen Rieselturm, der Füllkörper enthält. Über die Füllung läßt man den Aldehyd herabrieseln und leitet in den Turm gleichzeitig im Gleich- oder Gegenstrom Sauerstoff oder ein Sauerstoff enthaltendes Gasgemisch ein.

Die gewünschte Bicyclo[4.3.0]nonan-3(4),7(8)-dicarbonsäure fällt nach Entfernung des Lösungsmittels als Feststoff an, der durch geeignete Maßnahmen, beispielsweise durch Umsalzen und Umkristallisation weiter aufgereinigt werden kann.

In einer weiteren Ausgestaltung des erfindungsgemäßen Verfahrens wird zunächst 3(4),7(8)-Bisformyl-bicyclo[4.3.0]nonan zum 3(4),7(8)-Dihydroxymethyl-bicyclo[4.3.0]nonan hydriert und anschließend in einer Alkalischmelze bei erhöhter Temperatur und erhöhtem Druck umgesetzt.

Die Hydrierung des rohen oder gegebenenfalls aufgereinigten 3(4),7(8)-Bisformyl-bicyclo[4.3.0]nonan zum 3(4),7(8)-Dihydroxymethylbicyclo[4.3.0]nonan erfolgt unter allgemein üblichen Reaktionsbedingungen in Gegenwart konventioneller Hydrierkatalysatoren. Im allgemeinen beträgt die Hydriertemperatur 70 bis 170°C und der angewandte Druck 1 bis 30 MPa. Als Hydrierkatalysatoren sind besonders Nickelkatalysatoren geeignet.

Das katalytisch aktive Metall kann auf einem Träger aufgebracht werden, im allgemeinen in einer Menge von etwa 5 bis etwa 70 Gew.-%, vorzugsweise etwa 10 bis etwa 65 Gew.-% und insbesondere etwa 20 bis etwa 60 Gew.-% jeweils bezogen auf das Gesamtgewicht des Katalysators. Als Katalysatorträger eignen sich alle herkömmlichen Trägermaterialien, z.B. Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten Nickel und Trägermaterial können die Katalysatoren noch Zusatzstoffe in untergeordneten Mengen enthalten, die z.B. der Verbesserung ihrer Hydrieraktivität und/oder ihrer Standzeit und/oder ihrer Selektivität dienen. Derartige Zusatzstoffe sind bekannt, zu ihnen gehören z.B. die Oxide des Natriums, Kaliums, Magnesiums, Calciums, Bariums, Zinks, Aluminiums, Zirconiums und Chroms. Sie werden dem Katalysator im allgemeinen in einem Anteil von insgesamt 0,1 bis 50 Gew.-Teile bezogen auf 100 Gew.-Teile Nickel zugesetzt.

Aber auch trägerfreie Katalysatoren, wie Raney-Nickel oder Raney-Kobalt können in der Hydrierstufe verwendet werden.

Die Hydrierstufe wird diskontinuierlich oder kontinuierlich in flüssiger Phase mit suspendierten Katalysatoren oder in flüssiger oder gasförmiger Phase mit fest angeordneten Katalysatoren durchgeführt; die kontinuierliche Arbeitsweise wird bevorzugt.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten. In jedem Fall ist dafür Sorge zu tragen, dass das Hydriergas frei von Katalysatorgiften wie Schwefelverbindungen oder Kohlenmonoxid in schädlichen Mengen ist.

Das so erhaltene Diol wird anschließend zweckmäßigerweise destillativ gereinigt und anschließend in einer Alkalischmelze bei erhöhter Temperatur, im allgemeinen von 220 bis 350°C und bei erhöhtem Druck, im allgemeinen von 0,5 bis 5 MPa, umgesetzt. Zur Herstellung der Alkalischmelze kommen Alkalimetallhydroxide, wie Natriumhydroxid oder Kaliumhydroxid, sowie deren Mischungen zum Einsatz. Üblicherweise beträgt das Gewichtsverhältnis von Diol zu Alkalimetallhydroxid 1 : 2,1 bis 1 : 2,6 vorzugsweise 1 : 2,1 bis 1 : 2,3 Während der Umsetzung kommt es zur Freisetzung von Wasserstoff, so dass die Reaktion vorzugsweise in einem geschlossenen Druckbehälter durchgeführt wird. Zweckmäßigerweise arbeitet man in Abwesenheit von Lösungsmitteln, obwohl die Umsetzung auch in Gegenwart hochsiedender, inerter Lösungsmittel durchgeführt werden kann. Nach beendeter Reaktion wird das Reaktionsgemisch mit Wasser versetzt und neutrale Nebenprodukte können mit einem organischen Lösungsmittel, wie beispielsweise Toluol, extrahiert werden. Die Wasserphase wird anschließend angesäuert und die freigesetzte Dicarbonsäure in der anfallenden organischen Phase abgetrennt. Auch hier ist der Einsatz eines geeigneten organischen Lösungsmittels möglich. Falls notwendig kann die so hergestellte Dicarbonsäure bei vermindertem Druck von dem verwendeten organischen Lösungsmittel befreit oder durch Umkristallisation weiter aufgereinigt werden.

Das erfindungsgemäße Verfahren gestattet einen einfachen und kostengünstigen Zugang zu Bicyclo[4.3.0]nonan-3(4),7(8)-dicarbonsäure in hoher Ausbeute und in hoher Reinheit. Die nach dem erfindungsgemäßen Verfahren hergestellte Dicarbonsäure lässt sich in ausgezeichneter Weise für unterschiedliche Anwendungen einsetzen, beispielsweise als Bestandteil in Lack- und Kunstharzformulierungen, für Weichmacher und in Schmierölen.

Im Folgenden wird das erfindungsgemäße Verfahren anhand einiger Beispiele näher erläutert, es ist jedoch nicht auf die beschriebenen Ausführungsformen beschränkt.

### Beispiele

### Herstellung von Bicyclo[4.3.0]nonan-3(4),7(8)-dicarbonsäure

### 1. Herstellung von 3(4),7(8)-Bisformyl-bicyclof4.3.Olnonan

In einem Stahlautoklaven mit Magnetrührer wurden 1000 g Bicyclo[4.3.0]nona-3,7-dien, technische Qualität, und 1000 g Toluol vorgelegt. Nach Zusatz von 12,75 g Triphenylphosphin sowie 50 mg Rh, in Form einer toluolischen Lösung von Rh-2-ethylhexanoat mit einem Gehalt von 7062 mg Rh/kg, wurde das Gemisch auf 130°C erwärmt und unter einem Druck von 26 MPa mit Synthesegas behandelt. Nach einer Reaktionszeit von 8 Stunden wurde die Hydroformylierungsreaktion beendet.

Die organische Phase wurde gaschromatographisch untersucht.

**GC-Analyse (Flächenprozent ohne Toluol)**

| | |
|---|---|
| Vorlaufkomponenten | 0,2 |
| Bicyclo[4.3.0]nona-3,7-dien-Bereich | 0,1 |
| Komponenten | 4,6 |
| 3(4),7(8)-Bisformyl-bicyclo[4.3.0]nonan | 89,1 |
| Triphenylphosphin/Triphenylphosphinoxid | 1,2 |
| Hochsieder | 4,8 |

### 2. Herstellung von Bicyclo[4.3.0]nonan-3(4),7(8)-dicarbonsäure

### 2.1 Alkalischmelze von 3(4),7(8)-Dihydroxymethyl-bicyclo[4.3.0]nonan

### 2.1.1 Herstellung von 3(4),7(8)-Dihydroxymethyl-bicyclo[4.3.0]nonan

Das nach der Hydroformylierung erhaltene rohe 3(4),7(8)-Bisformylbicyclo[4.3.0]nonan wurde durch Destillation an einem Dünnschichtverdampfer weitgehend vom Toluol befreit (Manteltemperatur 140°C, Druck 100 hPa). Es fiel ein Rückstand an, der, nach gaschromatographischer Analyse, neben 6,7 % Toluol und 83,8 % 3(4),7(8)-Bisformyl-bicyclo[4.3.0]nonan noch 9,5 % Komponenten enthielt.

Nachfolgend wurden 700 g des Rückstands, verdünnt mit 300 g Isobutanol, und 42 g Ni 52/35-Katalysator der Firma Johnson Matthey Plc - in einem 3-I Autoklav vorgelegt. Das Reaktionsgemisch wurde auf 130°C erwärmt und bei einem Druck von 10,0 MPa und einer Reaktionszeit von 8 Stunden umgesetzt. Nach Reaktionsende wurde abgekühlt, entspannt und vom Katalysator abfiltriert. Das so erhaltene Reaktionsprodukt wurde gaschromatographisch untersucht.

**GC-Analyse (Flächenprozent)**

| | |
|---|---|
| Vorlaufkomponenten | 1,3 % |
| Isobutanol / Toluol / Methylcyclohexan | 29,2 % |
| 3(4),7(8)-Dihydroxymethyl-bicyclo[4.3.0]nonan | 62,8 % |
| Sonstige | 6,7 % |

Zur Aufarbeitung wurde das rohe Hydrierprodukt an einer Claisenbrücke destilliert. Beim Einsatz von 825,3 g erhielt man 459,3 g Hauptfraktion in einem Siedebereich von 178 - 179°C bei einem Druck von 1 hPa mit folgender Zusammensetzung:

**GC-Analyse (Flächenprozent)**

| | |
|---|---|
| Vorlaufkomponenten | 0,1 % |
| 3(4),7(8)-Dihydroxymethyl-bicyclo[4.3.0]nonan | 97,3 % |
| Sonstige | 2,6 % |

### 2.1.2. Alkalischmelze von 3(4),7(8)-Dihydroxymethyl-bicyclo[4.3.0]nonan

Das nach der Hydrierung von 3(4),7(8)-Bisformyl-bicyclo[4.3.0]nonan und nachfolgender Destillation erhaltene 3(4),7(8)-Dihydroxymethylbicyclo[4.3.0]nonan wurde in die Alkalischmelze eingesetzt.

184,3 g des Diols wurden zusammen mit 12,0 g Essigsäure in einem 1 1-Autoklav vorgelegt und mit 57,6 g NaOH (fest, rein) sowie 62,6 g KOH (fest, 85 %ig) versetzt. Unter Rühren wurde das Gemisch auf 250°C erwärmt, wobei der Druck auf 2 MPa abgeregelt wurde. Nach 2 Stunden bei 250°C wurde die Temperatur auf 280°C erhöht, der Druck verblieb bei 2 MPa. Nach einer Reaktionszeit von 2 Stunden bei 280°C wurde das Gemisch abgekühlt, wobei bei etwa 160°C Wasser in einer Menge von 300 g zugepumpt wurde. Die angefallene Lösung wurde mit 185,0 g Toluol versetzt und durch Zugabe von 1813,0 g 20 %iger wässriger Schwefelsäure angesäuert. Die organische Phase wurde anschließend fünfmal mit 300,0 g Wasser extrahiert. Nach Abdestillation des Lösungsmittels fiel die gewünschte Dicarbonsäure im Destillationsrückstand als Feststoff an. Die Auswaage an Bicyclo[4.3.0]nonan-3(4),7(8)-dicarbonsäure betrug 197,1g bei einer Reinheit von 99,07 %.

**Charakterisierung:**

| Elementaranalyse | |
|---|---|
| C₁₁H₁₆O₄ (212,2) | Ber. C 62,3 %, H 7,6 %, O 30,2 % |
| | Gef. C 63,3 %, H 7,2 %, O 27,7 % |

NMR-Daten
¹H-NMR (500 MHz, DMSO-d₆, ppm): 1,06-2,88 (m, 14 H, CH und CH₂), 11,95 (s, 2H, COOH)
¹³C-NMR (125 MHz, DMSO-d₆, ppm): 21,02-49,24 (CH und CH₂), 174,64-177,97 (COOH)
IR-Daten (Diamant-ATR-IR Spektroskopie)
ν (cm⁻¹) 3000 (m, br), 2933 (m), 2865 (m), 1691 (s), 926 (m)

### 2.2. Oxidation von 3(4),7(8)-Bisformvl-bicvclo[4.3.0]nonan

Die Flüssigphasenoxidation von 3(4),7(8)-Bisformyl-bicyclo[4.3.0]nonan zu Bicyclo[4.3.0]nonan-3(4),7(8)-dicarbonsäure wurde ohne Katalysatorzusatz in einem Blasensäulenreaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur damit konstant gehalten. Die Sauerstoffzufuhr erfolgte von unten durch eine mit dem Blasensäulenreaktor verbundene Glasfilterplatte mit einer maximalen Porenweite von 16-40 µm.

In die Oxidation wurden 467,8 g Dialdehyd mit einem Gehalt von 15,6 % Toluol, 71,4 % 3(4),7(8)-Bisformyl-bicyclo[4.3.0]nonan sowie 13,0 % sonstigen Komponenten eingesetzt, der nach Lösungsmittelabtrennung an einem Dünnschichtverdampfer gemäß Beispiel 2.1.1 anfiel. Gemischt wurde das Produkt mit 322,0 g Toluol sowie mit 42,5 g einer Lösung, bestehend aus Kalium-2-ethylhexanoat und 2-Ethylhexansäure mit einem Kaliumgehalt von 5,7 %.

Nach einer Reaktionszeit von 6 Stunden bei konstant 60°C wurde eine Rohsäure der folgenden Zusammensetzung erhalten:

**GC-Analyse (Flächenprozent ohne Toluol und 2-Ethylhexansäure):**

| | |
|---|---|
| 3(4),7(8)-Bisformyl-tricyclo[4.3.0]nonan | 4,7 % |
| Bicyclo[4.3.0]nonan-3(4),7(8)-dicarbonsäure | 75,5 % |
| Sonstige | 19,8 % |

Das erfindungsgemäße Verfahren eröffnet einen eleganten Herstellungsweg für Bicyclo[4.3.0]nonan-3(4),7(8)-dicarbonsäure in hohen Ausbeuten. Die neue Verbindung Bicyclo[4.3.0]nonan-3(4),7(8)-dicarbonsäure weist eine alicyclische Ringstruktur mit kondensierten Ringen auf, die sich hervorragend als Bestandteil für Lack- und Kunstharzformulierungen eignet.

## Patentansprüche

1. Verfahren zur Herstellung von Bicyclo[4.3.0]nonan-3(4),7(8)-dicarbonsäure durch Hydroformylierung von Bicyclo[4.3.0]nona-3,7-dien mit nachfolgender Oxidation, **dadurch gekennzeichnet, dass** man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160 °C und Drücken von 5 bis 35 MPa mit Synthesegas umsetzt und das so erhaltene 3(4),7(8)-Bisformyl-bicyclo[4.3.0]nonan anschließend zur Bicyclo[4.3.0]nonan-3(4),7(8)-dicarbonsäure oxidiert.

2. Verfahren zur Herstellung von Bicyclo[4.3.0]nonan-3(4),7(8)-dicarbonsäure durch Hydroformylierung von Bicyclo[4.3.0]nona-3,7-dien mit nachfolgender Hydrierung und anschließender Umsetzung in einer Alkalischmelze, **dadurch gekennzeichnet, dass** man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70°C bis 160°C und Drücken von 5 bis 35 MPa mit Synthesegas umsetzt, das so erhaltene 3(4),7(8)-Bisformyl-bicyclo[4.3.0]nonan zum 3(4),7(8)-Dihydroxymethyl-bicyclo[4.3.0]nonan hydriert, das so erhaltene 3(4),7(8)-Dihydroxymethyl-bicyclo[4.3.0]nonan in einer Alkalischmelze bei erhöhter Temperatur und erhöhtem Druck umsetzt und anschließend Bicyclo[4.3.0]nonan-3(4),7(8)-dicarbonsäure freisetzt.

3. Verfahren gemäß Anspruch 1 oder 2, **dadurch gekennzeichnet, dass** man als Organophosphorverbindungen organische Phosphor(III)-Verbindungen ausgewählt aus der Gruppe von Triarylphosphinen, Trialkylphosphinen, Alkylarylphosphinen, Alkylphosphiten, Arylphosphiten, Alkyldiphosphiten oder Aryldiphosphiten einsetzt.

4. Verfahren nach Anspruch 3, **dadurch gekennzeichnet, dass** man als Triarylphosphin Triphenylphosphin und als Arylphosphit Tris(2-tertiärbutylphenyl)phosphit oder Tris(2-tertiär-butyl-4-methylphenyl)phosphit einsetzt.

5. Verfahren gemäß einem oder mehreren der Ansprüche 1 bis 4, **dadurch gekennzeichnet, dass** als Übergangsmetallverbindungen der Gruppe VIII des Periodensystems der Elemente Verbindungen von Rhodium, Kobalt, Iridium, Nickel, Palladium, Platin, Eisen oder Ruthenium verwendet werden.

6. Verfahren gemäß einem oder mehreren der Ansprüche 1 bis 5, **dadurch gekennzeichnet, dass** als Übergangsmetallverbindungen der Gruppe VIII des Periodensystems der Elemente Verbindungen von Rhodium eingesetzt werden.

7. Verfahren gemäß einem oder mehreren der Ansprüche 1 bis 6, **dadurch gekennzeichnet, dass** man Rhodium in einer Konzentration von 5 bis 1000 Gew.-ppm, bezogen auf das homogene Reaktionsgemisch einsetzt.

8. Verfahren gemäß Anspruch 7, **dadurch gekennzeichnet, dass** man Rhodium in einer Konzentration von 10 bis 700 Gew.-ppm, vorzugsweise von 20 bis 500 Gew.-ppm, bezogen auf das homogene Reaktionsgemisch, einsetzt.

9. Verfahren gemäß einem oder mehreren der Ansprüche 1 bis 8, **dadurch gekennzeichnet, dass** das molare Verhältnis von Rhodium zu Phosphor 1 : 5 bis 1 : 200 beträgt.

10. Verfahren gemäß Anspruch 9, **dadurch gekennzeichnet, dass** das molare Verhältnis von Rhodium zu Phosphor 1 : 10 bis 1: 100 beträgt.

11. Verfahren gemäß einem oder mehreren der Ansprüche 1 bis 10, **dadurch gekennzeichnet, dass** man bei der Hydroformylierung bei Temperaturen von 80 bis 150°C, vorzugsweise von 90 bis 140°C, und bei Drücken von 10 bis 30 MPa, vorzugsweise von 20 bis 30 MPa, arbeitet.

12. Verfahren gemäß einem oder mehreren der Ansprüche 2 bis 11, **dadurch gekennzeichnet, dass** man die Hydrierung in Gegenwart von Nickelkatalysatoren bei Temperaturen von 70 bis 170°C und bei Drücken von 1 bis 30 MPa durchführt.

13. Verfahren gemäß einem oder mehreren der Ansprüche 2 bis 11, **dadurch gekennzeichnet, dass** man 3(4),7(8)-Dihydroxymethylbicyclo[4.3.0]nonan in einer Alkalischmelze bei Temperaturen von 220 bis 350°C und bei Drücken von 0,5 bis 5 MPa umsetzt.

14. Verfahren gemäß einem oder mehreren der Ansprüche 1, 3 bis 11, **dadurch gekennzeichnet, dass** man die Oxidation von 3(4),7(8)-Bisformyl-bicyclo[4.3.0]nonan bei Temperaturen von 20 bis 80°C und bei Drücken von Normaldruck bis 1,0 MPa durchführt.

15. Verfahren nach Anspruch 14, **dadurch gekennzeichnet, dass** man als Oxidationsmittel Sauerstoff oder Luft verwendet.

16. Die chemische Verbindung Bicyclo[4.3.0]nonan-3(4),7(8)-dicarbonsäure.
